# EUROPEAN PATENT APPLICATION

(11) **EP 1 416 417 A2**
(43) Date of publication of application: **06.05.2004**
(21) Application number: 03022230.1
(22) Date of filing: 01.10.2003
(51) Int. Cl.: G06F 19/00

(54) **Mehtod and systems for data management in patient diagnoses and treatment**

(30) Priority: 08.10.2002 US 416678 P
(71) Applicant: Bayer HealthCare, LLC, Tarrytown, New York 10591 (US)
(72) Inventor: Huang, Dijia, Granger IN 46530 (US)
(74) Representative: Linhart, Angela

(57) **Abstract**

A method and clinical analyzer system are provided for implementing data management to aid analysis and treatment. The clinical analyzer system includes a biosensor for receiving and processing a user sample and a memory for means for storing a plurality of patient records including predefined parameter data values. A processor device is coupled to the biosensor for receiving and processing the plurality of patient records, and generating a report. A fax driver coupled to the processor device sends the generating report via a telephone network to a predefined fax machine at a physician's office.

## Description

### Field of the Invention

The present invention generally relates to a clinical analyzer, and, more particularly, to new and improved methods and systems for implementing data management in patient diagnoses and treatment.

### Description of the Prior Art

The quantitative determination of analytes in body fluids is of great importance in the diagnoses and maintenance of certain physiological abnormalities. For example lactate, cholesterol and bilirubin should be monitored in certain individuals. In particular, the determination of glucose in body fluids is of great importance to diabetic individuals who must frequently check the level of glucose in their body fluids as a means of regulating the glucose intake in their diets. While the remainder of the disclosure herein will be directed towards the determination of glucose, it is to be understood that the procedure and apparatus of this invention can be used with other diagnostic systems.

Home glucose monitoring by diabetics is becoming increasingly routine in modern-day diabetes management. Historically patients were required to maintain hand-written paper log books for manually recording glucose readings and other relevant information. More specifically, patients measured their blood glucose at scheduled times, and recorded this information in a personal log book.

Current diagnostic systems, such as, blood glucose systems include a biosensor used to calculate the actual glucose value based on a measured output and the known reactivity of the reagent sensing element used to perform the test. The test results typically are displayed to the user and stored in a memory in the blood glucose monitor. In some known systems, the multiple stored values from the blood glucose monitor are periodically transferred to a separate computer, for example to enable analysis by a doctor for the blood glucose monitor user.

While the introduction of glucose meters with various memory functions has greatly simplified the data recording process and increased the reliability of stored data, the large amounts of recorded data have made the interpretation task complicated. It is also possible with present-day devices for patients to record other clinically relevant data such as diet and exercise factors, and life-style information. All such stored data can conveniently be transferred to a physician's office, typically via a communications link such as an acoustic modem line, where it can be reviewed in printed or displayed for making appropriate treatment recommendations.

Many traditional approaches to automated analysis of diabetes data provide a relatively superficial analysis and an assortment of graphical displays based upon certain predefined statistical calculations. However, the time-consuming and complicated synthesis and interpretation of clinical implications associated with the processed data still need to be performed by the reviewing physician, and significant interaction is still required on behalf of the physician.

U.S. patent 5,251,126 issued October 5, 1993 to Kahn et al., and assigned to the present assignee discloses an automated diabetes data interpretation method referred to as the "IDDI" system, that combines symbolic and numeric computing approaches in order to identify and highlight key clinical findings in the patient's self-recorded diabetes data. The patient data, including blood glucose levels and insulin dosage levels, recorded by a diabetic patient over a period of time by means of a glucose meter or the like, is initially downloaded into a central processing system such as a personal computer. The accepted diabetes data is processed to (a) identify insulin dosage regimens corresponding to predefined significant changes in insulin dosage which are found to be sustained for at least a predefined segment of the overall data collection period, (b) identify statistically significant changes in blood glucose levels resulting across adjacent ones of the identified insulin regimen periods, and (c) identify clinically significant changes in blood glucose levels from within the identified statistically significant glucose level changes. The results of the diabetes data processing are generated in the form of a comprehensive yet easily understandable data interpretation report highlighting the processing results, including details pertaining to the identified insulin regimens and the associated clinically significant changes in glucose levels.

Multiple commercially available clinical analyzers are available for patient use. Due to differences between various commercially available clinical analyzers, a health care professional (HCP) must have compatible software to run, or may require the patient to be present in the HCP's office if the patient does not have the same or similar program at home. The HCP must run the program, switch cables to match the meter, and maintain both hardware and software. Such chores tend to be time consuming and inefficient.

A need exists for an improved method and clinical analyzer system for implementing data management to aid analysis and treatment by the patient's doctor or HCP and to minimize time required, for example, in running software, switching cables, and downloading meters.

### Summary of the Invention

Important objects of the present invention are to provide a new and improved method and clinical analyzer system for implementing data management to aid analysis and treatment; to provide such method and apparatus that eliminates or minimizes the need for user interaction; and to provide such method and apparatus that overcome some disadvantages of prior art arrangements.

In brief, a method and clinical analyzer system are provided for implementing data management to aid analysis and treatment. The clinical analyzer system includes a biosensor for receiving and processing a user sample and a memory for means for storing a plurality of patient records including predefined parameter data values. A processor device is coupled to the biosensor for receiving and processing the plurality of patient records, and generating a report. A fax driver coupled to the processor device sends the generating report via a telephone network to a predefined fax machine at a physician's office.

In accordance with features of the invention, the processor device is a handheld computer device, such as a handheld personal data assistant (PDA). Alternatively, the plurality of patient records can be transferred to a server computer that processes the plurality of patient records, generates and faxes the report to the predefined fax machine at a physician's office.

### Brief Description of the Drawings

The present invention together with the above and other objects and advantages may best be understood from the following detailed description of the preferred embodiments of the invention illustrated in the drawings, wherein:
FIG. 1 is a logical block diagram representation of a first clinical analyzer system for implementing data management in patient diagnoses and treatment including a central server in accordance with the present invention;
FIG. 2 is a logical block diagram representation of a second clinical analyzer system for implementing data management in patient diagnoses and treatment without a central server in accordance with the present invention;
FIGS. 3-4 are flow charts respectively illustrating exemplary sequential steps of using the clinical analyzer systems of FIGS. 1 and 2 with the data management methods in accordance with the present invention.

### Detailed Description of the Preferred Embodiments

In accordance with features of the invention, a new and improved method and clinical analyzer systems are provided for implementing data management to aid analysis and treatment report that eliminates or minimizes the need for user interaction. In the physician's office, only a standard facsimile or fax machine is required by the method and clinical analyzer systems of the invention. Automated intelligent diabetes data interpretation (IDDI) software processes, analyzes and interprets recorded diabetes patient data and generates a report that is faxed to the physician's office. User interaction by the patient is not required in the data processing, report generation or faxing the generated report to the physician's office. The physician receives the faxed report that is ready to be entered into the patient's file without requiring data processing or interaction by the physician. The methods and clinical analyzer systems of the invention eliminate the need for a patient to download recorded diabetes patient data to the physician's office and eliminate the need for the physician to have any computer or data processing system for processing, analyzing and interpreting recorded diabetes patient data.

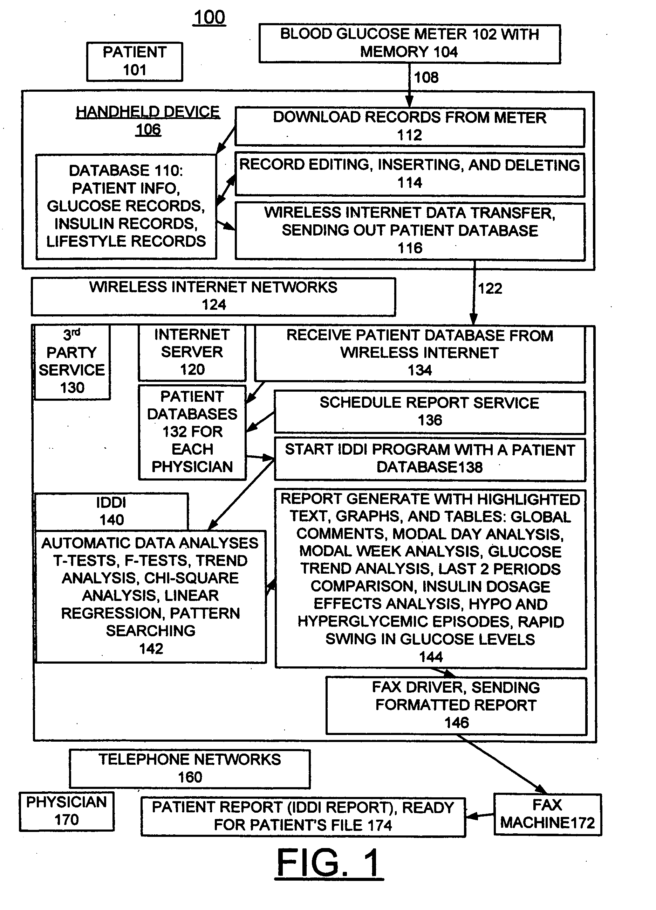

Having reference now to the drawings, in FIG. 1 there is illustrated a clinical analyzer system designated as a whole by the reference character 100 and arranged in accordance with principles of the present invention. Clinical analyzer system 100 includes a biosensor or glucose meter 102 used by a patient indicated as 101. The glucose meter 102 periodically receives and processes a user sample from the patient 101. A memory 104 is included in the glucose meter 102 for storing or recording the measured blood glucose (BG) levels. Clinical analyzer system 100 includes a handheld processor device or personal data assistant 106, such as a Palm™ handheld personal data assistant, coupled to the glucose meter 102 through an appropriate communication link 108, such as a direct connection between the meter 102 and handheld processor device 106.

Handheld processor device 106 is used by the patient together with the meter 102 to download records from the meter, and to also allow the user to augment stored glucose results by entering and storing insulin dosage records and time as well as other relevant markers, for example, lifestyle markers such as for diet, exercise, symptoms, and the like, during a given monitoring period that are stored in a patient database 110. As indicated in a block 112, handheld device 106 includes a function to download records from the meter 102. Handheld device 106 includes a function to allow the user to edit, insert and delete records maintained in the user database 110 as indicated in a block 114. Handheld device 106 includes a patient database transfer function as indicated in a block 116 to transfer data stored within the patient database 110 to an Internet server computer 120.

Internet server computer 120 of the clinical analyzer system 100 is a central server computer coupled to each of multiple handheld processor devices 106 of respective patients 101 via a respective Internet connection 122 (one shown) within wireless Internet networks 124. A third party service indicated as 130 maintains the Internet server computer 120 and can charge a subscribe fee to participating physicians. Internet server computer 120 stores a plurality of patient databases 132 for each physician. Internet server computer 120 includes a function as indicated in a block 134 to receive patient data 110 from a wireless Internet connection 122. Internet server computer 120 includes a function as indicated in a block 136 for scheduling a report service for each received patient database 132.

Internet server computer 120 includes a function as indicated in a block 138 to start an IDDI program 140 for a particular patient database 132. Internet server computer 120 includes the automated intelligent diabetes data interpretation (IDDI) software 140 necessary to process, analyze and interpret the self-recorded diabetes patient data as indicated in a block 142 in accordance with predefined flow sequences and generate an appropriate data interpretation output report as indicated in a block 144.

A given patient database or data file is processed by the server computer 120 in accordance with the IDDI system software 140 in such a manner as to extract clinically meaningful information that is presented in a comprehensive and informative report. The report 144 is particularly adapted for convenient use by a physician toward arriving at meaningful or intelligent clinical and/or theràpeutic decisions. The data interpretation report 144 is comprehensive and replaces the laborious paging through and manual review by a physician of the inordinately large and difficult to comprehend amount of raw data contained in the patient log. It should be noted that the IDDI system software 140 requires no user intervention once the database to be interpreted is available in the form of a patient database in system memory 132. The IDDI report 144 is contains, for example, highlighted text, graphs, and tables global comments, modal day analysis, modal week analysis, last 2 periods comparison, insulin dosage effects analysis, hypo and hyperglycemic episodes, rapid swing in glucose levels, and the like.

The IDDI system software 140 uses a combination of symbolic and numerical methods to analyze the data, detect clinical implications contained in the data and present the pertinent information in the form of a graphics-based data interpretation report 144. The symbolic methods used by the IDDI system encode the logical methodology used by expert diabetologists as they examine patient logs for clinically-significant findings, while the numeric or statistical methods test the patient data for evidence to support a hypothesis posited by the symbolic methods which may be of assistance to a reviewing physician.

U.S. patent 5,251,126 discloses an IDDI system that advantageously included in the IDDI software 140 in the Internet server computer 120. The subject matter of the above-identified U.S. patent 5,251,126 is incorporated herein by reference.

Server computer 120 includes a fax driver 146 coupled to telephone networks 160 for sending the formatted report to a particular physician 170 with a fax machine 172 at the patient's physician office. An IDDI patient report is printed that is ready for the patient's file as indicated in a block 174.

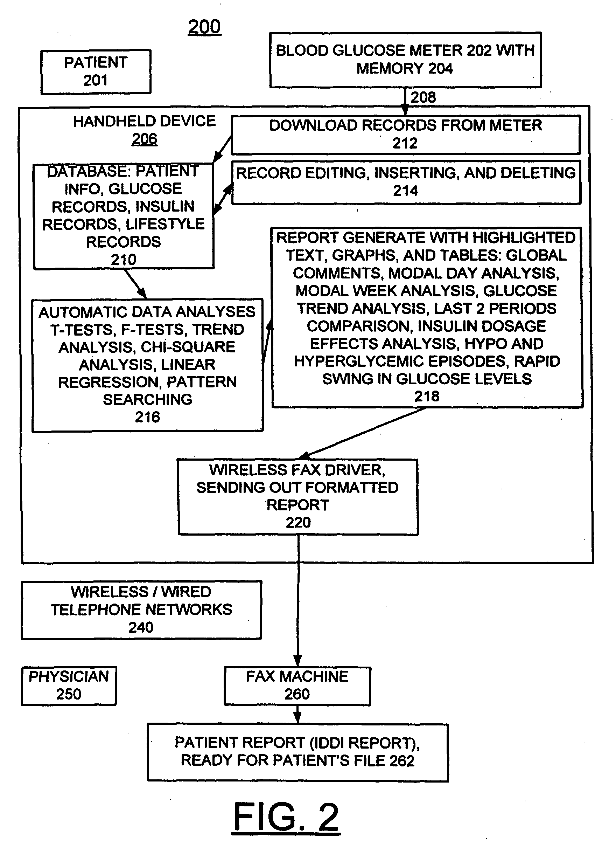

Referring now to FIG. 2, there is shown a second clinical analyzer system for implementing data management in patient diagnoses and treatment without a central server designated as a whole by the reference character 200 in accordance with the present invention. Clinical analyzer system 200 similarly includes a biosensor or glucose meter 202 used by a patient for receiving and processing a user sample and the meter includes a memory 204 for storing predefined parameter data values or recording measured blood glucose (BG) levels. Clinical analyzer system 200 includes a handheld processor device or personal data assistant (PDA) 206, such as a Palm™ handheld personal data assistant or a cell phone with PDA functions.

Handheld processor device 206 is used by the patient together with the meter 202 to download records from the meter, and to also allow the user to augment stored glucose results by entering and storing insulin dosage records and time as well as other relevant markers, for example, lifestyle markers such as for diet, exercise, symptoms, and the like, during a given monitoring period. Handheld device 206 allows the user to edit, insert and delete records maintained in a user database 210. Data stored within the glucose meter 202 is transferred through an appropriate communication link 208, such as a direct connection between the meter 202 and handheld device 206.

Handheld processor device 206 includes automated intelligent diabetes data interpretation (IDDI) software 216 necessary to process, analyze and interpret the self-recorded diabetes patient data in accordance with predefined flow sequences and generate an appropriate data interpretation output. The IDDI system of U.S. patent 5,251,126 as included in the IDDI software 120 of system 100 in FIG. 1, advantageously is used to implement the IDDI software 216 in the system 200. An IDDI report 218 is generated that contains, for example, highlighted text, graphs, and tables global comments, modal day analysis, modal week analysis, last 2 periods comparison, insulin dosage effects analysis, hypo and hyperglycemic episodes, rapid swing in glucose levels, and the like.

As compared to system 100 of FIG. 1, the clinical analyzer system 200 eliminates the server computer 120 with the handheld processor device 206 performing the automatic data analyses and generating an appropriate report. Handheld processor device 206 includes a fax driver 220 coupled to telephone networks 240 for sending a physician 250 the formatted report 218 via a fax machine 260 at the patient's physician office. An IDDI patient report 262 is printed that is ready for the patient's file.

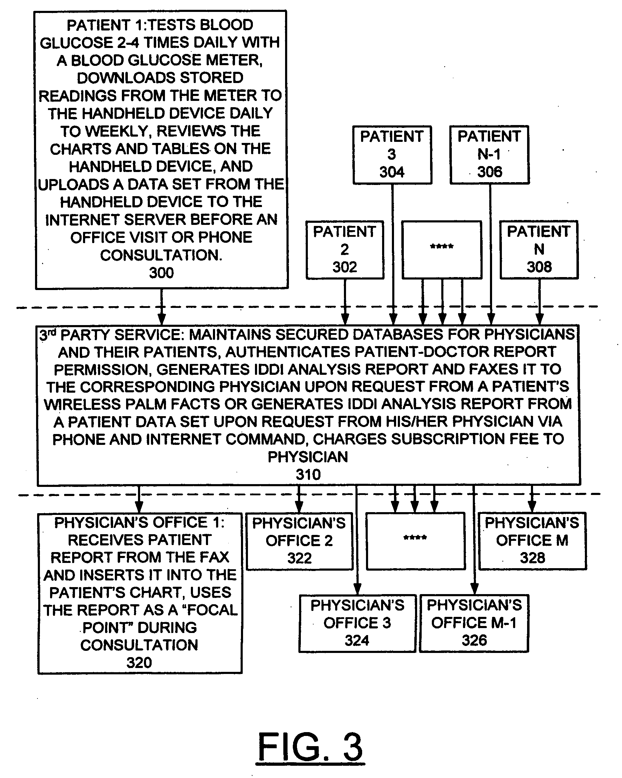

Referring to FIG. 3, there are shown exemplary sequential operations using the clinical analyzer system 100 in accordance with the data management methods of the present invention. As indicated in a block 300, a patient tests blood glucose levels, for example, 2-4 times daily with a blood glucose meter 102; downloads stored readings from the meter memory 104 to the handheld device 106 daily to weekly; reviews charts and tables on the handheld device 106; and transfers or uploads a data set from the handheld device 106 to the Internet server 120, for example, before an office visit or phone consultation with the physician. Multiple patients 1-N, 300, 302, 304, 306 and 308 record diabetes patient data and transfer data sets from handheld devices 106 to the Internet server 120.

As indicated in a block 310, the third party service 130 maintains the Internet server computer 120 that maintains secured databases for participating physicians and their patients, authenticates patient-doctor report permission; generates IDDI analysis report and faxes the generated report to the corresponding physician upon request from a patient handheld device 106; or generates IDDI analysis report and faxes the generated IDDI report to the corresponding physician upon request from the patient's physician via phone or Internet command; and charges a subscription fee to the physician.

As indicated in a block 320, a physician's office receives a patient report from the fax sent by the Internet server computer 120, and inserts the faxed report into the patient's chart. The physician uses the report as a focal point during consultation with the patient. Multiple physicians' offices 1-M 320, 322, 324, 326 and 328 receive faxed IDDI reports from the Internet server 120.

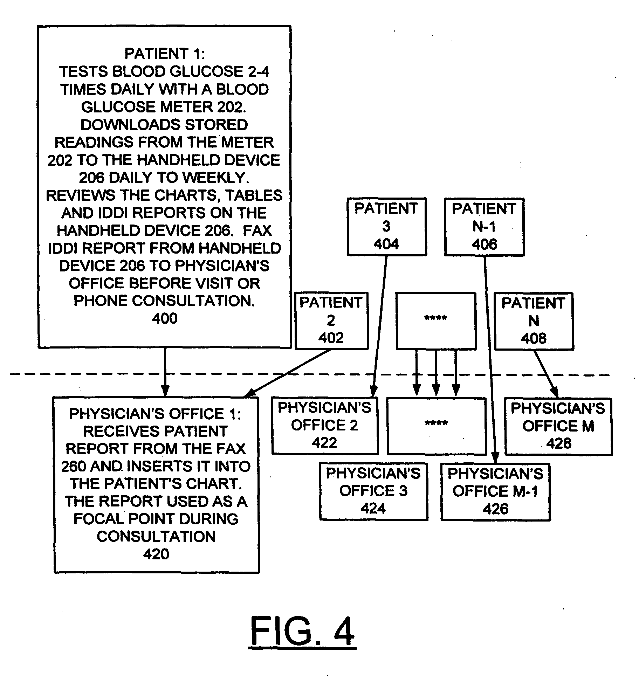

Referring to FIG. 4, there are shown exemplary sequential operations using the clinical analyzer system 200 in accordance with the data management methods of the present invention. As indicated in a block 300, a first patient tests blood glucose levels, for example, 2-4 times daily with a blood glucose meter 202; downloads stored readings from the meter memory 204 to the handheld device 206 daily to weekly; reviews charts, tables and IDDI reports on the handheld device 206; and faxes the generated IDDI report 218 to the patient's physician's office, for example, before consultation with the physician. Multiple patients 1-N, 400, 402, 404, 406 and 408, using the meter 202 and handheld device 206, record, process, analyze diabetes patient data and generate IDDI reports that are faxed to the patients' physician's offices before an office visit or telephone consultation.

As indicated in a block 420, the physician's office receives a patient report from the fax sent by a respective patient's handheld device 206, and inserts the faxed report into the patient's chart. The physician uses the report as a focal point during consultation with the patient. Multiple physicians' offices 1-M 420, 422, 424, 426 and 428 receive faxed IDDI reports from respective patients' handheld devices 206.

Handheld processor devices 106, 206 can be implemented using any suitable computer, such as the Palm™ personal data assistant (PDA) or similar devices. Internet server 120 can be implemented using any suitable server such as the AS/400@ computer system, running the OS/400® operating system, both products of International Business Machines Corporation, located in Armonk, New York. Handheld processor device 106 and Internet server 120 could be other types of computer systems, whether they be microcomputers such as an Apple Macintosh or mainframe computers such as an IBM System/390, and still fall within the spirit and scope of this invention. It should be understood that the invention is not limited to the particular hardware designs, software designs, communications protocols, performance parameters, or application-specific functions.

While the present invention has been described with reference to the details of the embodiments of the invention shown in the drawings, these details are not intended to limit the scope of the invention as claimed in the appended claims.

## Claims

1. A clinical analyzer system for implementing data management to aid analysis and treatment comprising:
a biosensor for receiving and processing a user sample, said biosensor including memory means for storing a plurality of patient records including predefined parameter data values;
a processor device coupled to said biosensor for receiving and processing said plurality of patient records, and generating a report; and
a fax driver coupled to said processor device for sending said generated report via a telephone network to a predefined fax machine at a physician's office receiving said generating report.

2. A clinical analyzer system as recited in claim 1 wherein said processor device includes a handheld computer device.

3. A clinical analyzer system as recited in claim 2 wherein said handheld computer device includes a handheld personal data assistant.

4. A clinical analyzer system as recited in claim 1 wherein said processor device includes a server computer.

5. A clinical analyzer system as recited in claim 4 wherein said server computer includes an Internet server computer.

6. A clinical analyzer system as recited in claim 4 includes a handheld computer device coupled to said biosensor receiving patient records; and said handheld computer device coupled to said server computer for transferring a set of patient records to said server computer.

7. A clinical analyzer system as recited in claim 6 wherein said handheld computer device is coupled to said server computer via an Internet network connection.

8. A clinical analyzer system as recited in claim 1 wherein said processor device includes automated intelligent diabetes data interpretation (IDDI) processing software for generating a predefined IDDI report.

9. A clinical analyzer system as recited in claim 1 wherein said processor device includes a handheld cell phone with personal data assistant (PDA) functions.

10. A method for implementing data management to aid analysis and treatment comprising the steps of:
providing a biosensor for receiving a user sample and for performing a predefined test sequence for measuring a predefined parameter value;
storing patient records including said predefined parameter values in said biosensor, and
transferring stored data records from said biosensor to a computer device; said computer device performing the steps of:
processing said patient records and generating a report; and
sending said generated report via a telephone network to a predefined fax machine at a physician's office.

11. A method for implementing data management as recited in claim 10 wherein the step of transferring stored data records from said biosensor to a computer device includes the steps of downloading stored data records from said biosensor to a handheld computer device; and said handheld computer device performing the steps of processing said patient records and generating said report; and sending said generated report via said telephone network to said predefined fax machine at a physician's office.

12. A method for implementing data management as recited in claim 10 wherein the step of transferring stored data records from said biosensor to a computer device includes the steps of downloading stored data records from said biosensor to a handheld computer device; said handheld computer device further performing the steps of transferring said data records to a server computer.

13. A method for implementing data management as recited in claim 12 wherein the step of transferring said data records to a server computer includes the step of transferring said data records via an Internet network to said server computer.

14. A method for implementing data management as recited in claim 10 wherein the steps of processing said patient records and generating said report includes the steps of automated intelligent diabetes data interpretation (IDDI) processing said data records for generating a predefined IDDI report.
